(19) Europäisches Patentamt
European Patent Office
Office européen des brevets

(11) **EP 2 692 321 B1**

(12) **EUROPÄISCHE PATENTSCHRIFT**

(45) Veröffentlichungstag und Bekanntmachung des Hinweises auf die Patenterteilung:
**13.09.2017   Patentblatt 2017/37**

(51) Int Cl.:
*A61F 13/537* *(2006.01)*   *D04H 1/4258* *(2012.01)*
*D04H 1/435* *(2012.01)*   *D04H 1/485* *(2012.01)*

(21) Anmeldenummer: **13002677.6**

(22) Anmeldetag: **23.05.2013**

(54) **Zwischenspeichervlies**

Temporary storage non-woven fabric

Matelas d'accumulateur intermédiaire

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.08.2012   DE 102012015219**

(43) Veröffentlichungstag der Anmeldung:
**05.02.2014   Patentblatt 2014/06**

(73) Patentinhaber: **Sandler AG**
**95126 Schwarzenbach/Saale (DE)**

(72) Erfinder:
• **Die Erfinder haben auf ihr Recht verzichtet, als solche bekannt gemacht zu werden.**

(74) Vertreter: **Grünecker Patent- und Rechtsanwälte PartG mbB**
**Leopoldstraße 4**
**80802 München (DE)**

(56) Entgegenhaltungen:
**EP-A2- 0 397 110     EP-A2- 0 672 774**
**WO-A1-01/48291      WO-A1-98/02608**
**WO-A1-98/22065      WO-A1-98/22066**

EP 2 692 321 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft einen Aufnahme- und Verteilvliesstoff gemäß dem Oberbegriff des unabhängigen Anspruchs 1.

Bei Hygieneartikeln für die Aufnahme und Speicherung von Körperflüssigkeiten ist eine Grundforderung bei möglichst geringer Dicke des Gesamtaufbaus ein möglichst hohes Speichervermögen für aufzunehmende Körperflüssigkeiten zu erzielen.

In der Vergangenheit gab es verschiedene Ansätze, dies zu erreichen.

Prinzipiell haben die Aufbauten folgende Bestandteile

- Topsheet als zum Körper hingewandte Lage
- Aufnahme- und Verteillage zur raschen Aufnahme der auftreffenden Flüssigkeit und Verteilung über die gesamte Fläche des Hygieneproduktes.
- Speicherlage zur Immobilisierung der Flüssigkeitsmenge
- Backsheet als flüssigkeitsdichte Außenlage

In den letzten Jahren ging der Trend immer mehr zu einer Speicherlage, welche aus bis zu 100% superabsorbierenden Polymeren besteht. Die vorgeschaltete Aufnahme-und Verteillage hat daher die Aufgabe zum Einen, die Flüssigkeit zwischen zu speichern, um einem Gelblocking vorzubeugen, zum anderen muss die Flüssigkeit aber auch über möglichst die ganze Fläche des Hygieneartikels verteilt werden, sodass die Speicherschicht vollständig genutzt wird.

Es gab daher verschiedene Ansätze, die Kombination dieser beiden Funktionen zu erreichen:

Die DE 69222914 beschreibt einen zweilagigen Vliesstoff, bei welchem eine Lage hydrophobe, trotzdem wasserdurchlässige Eigenschaften aufweist, diese verteilt zunächst auftreffende Flüssigkeit und leitet diese an eine darunterliegende Schicht, die saugfähige, flüssigkeitsspeichernde Eigenschaften aufweist. Diese saugfähige Lage wird vorzugsweise aus Fasern auf cellulosischer Basis, beispielsweise Baumwolle oder Viskose gebildet, die hydrophobe Verteillage aus synthetischen Fasern wie Polypropylen oder Polyester.

Dieser mehrschichtige Aufbau hat den Nachteil der Grenzschichtenbildung am Übergang der hydrophoben Lage zur nachfolgenden flüssigkeitsspeichernden Schicht. Hier kommt es zu Beeinträchtigungen der Flüssigkeitsweiterleitung und somit zu hohen Run-Off-Zeiten. Gleiches gilt auch für nach der DE102006000780 hergestellte Vliese.

Andererseits gibt es gemäß der US4935022 den Ansatz mit sogenannten "crosslinked" Cellulosefasern, deren Nachteil allerdings die Verwendung zusätzlich eingebrachter Bindemittel ist, welche wiederum saugfähige Bestandteile inaktivieren. Des Weiteren kann es bei bindemittel- bzw. thermisch verfestigten Materialen nach der Quellung der Saugfaser zum Aufbrechen der Bindepunkte kommen.

Verwandter Stand der Technik ist bekannt aus EP0397110A2, WO98/22066 A1, WO98/02608 A1, WO01/48291 A1, EP0672774 A2, WO98/22065 A1, DE102007028039 A1, DE102008007804 A1, DE2226330A1, US20060258250 A1 und US2003087448 A1.

Aufgabe war es daher, ein Aufnahme- und Verteilvlies zu schaffen, welches die Nachteile des Standes der Technik vermeidet. Gelöst wird die Aufgabe anhand der Merkmale des Anspruches 1. Vorteilhafte Ausgestaltungen sind in den Ansprüchen 2-4 genannt.

Die Begriffe Saugfaser, Verteilfaser und Stützfaser sind wie folgt definiert:

Saugfaser: ist eine regenerierte Zellulosefaser, welche aus Lösungen von Zellulosederivaten hergestellt wird. Diese Viskosefaser kann eine Modifikation der Oberfläche haben und/oder des Querschnitts. Beispielsweise wird durch einen trilobalen Querschnitt die Saugleistung gegenüber rundem Querschnitt signifikant erhöht. Auch eine Viskose-Hohlfaser ist verwendbar.

Erfindungsgemäße Vertreter sind beispielsweise Viscostar-Viskosefasern oder normale Viskosefasern des Herstellers Lenzing, aber auch die sogenannte Galaxy-Faser der Fa. Kehlheim Fibers. Übliche Faserfeinheiten liegen im Bereich von 1,0 bis 3,3 dtex, bevorzugt 1,3 bis 2,2 dtex; die eingesetzten Faserlängen sind im Bereich von 10-70mm, bevorzugt 35-50mm.

Des Weiteren können Saugfasern auch aus regenerierten Zellulosefasern bestehen welche nach dem sogenannten Lyocell-Verfahren hergestellt werden und welche dadurch insbesondere im nassen Zustand eine bessere Stabilität aufweisen, so dass bei einer moderaten Flüssigkeitsaufnahme die Verteilung von Flüssigkeit begünstigt wird.

Erfindungsgemäße Vertreter sind beispielsweise Lyocell oder Tencel - Viskosefaser der Firma Lenzing. Übliche Faserfeinheiten liegen im Bereich von 1,0 bis 3,3 dtex, bevorzugt 1,3 bis 2,2 dtex; die eingesetzten Faserlängen sind im Bereich von 10-70mm, bevorzugt 35-50mm. Auch bei dem Einsatz der erwähnten Lyocell oder Tencel- Fasern können diese nicht vollständig die Aufgabe der Flüssigkeitsverteilung übernehmen, eine Beimengung von Polyester- und/oder Polyacrylfasern in Höhe eines Anteils von 20-80% innerhalb der eingesetzten Menge an Verteilfasern ist möglich.

Der Begriff "Verteilfaser" wird im Sinne der vorliegenden Erfindung für Faserstoffe aus thermoplastischen und/oder duroplastischen Polymeren verwendet, welche im erfindungsgemäßen Vliesstoff den Flüssigkeitstransport gewährleisten.

Zum Einsatz kommen dafür bevorzugt dauerhaft hydrophile Fasern aus thermoplastischen Polymeren wie Polyester oder Polypropylen. Geeignet sind aber auch duroplastische Polymere wie beispielsweise Polyacrylfasern.

Der Begriff hydrophil steht dabei für Fasern, welche im unverarbeiteten Zustand eine Sinkzeit von kleiner 5 Sekunden, bevorzugt von kleiner 3 Sekunden aufweisen. Die Ermittlung der Sinkzeit geschieht gemäß WSP10.1 (08), Abschnitt 7.1.

Neben Vollfasern, d.h. Fasern die über ihren Querschnitt vollständig aus Polymer bestehen, können auch Hohlfasern eingesetzt werden. Hohlfasern weisen über den Querschnitt der Faser ein oder mehrere Löcher auf, sodass derartige Fasern über ihre Länge gesehen einem Schlauch ähneln. Die Faserfeinheiten liegen im Bereich von 3,3 bis 17 dtex, bevorzugt 4,4 bis 10,0 dtex; die eingesetzten Faserlängen sind im Bereich von 10 - 80mm, bevorzugt 35-60mm. Weiterhin für die Flüssigkeitsverteilung eingesetzt werden Fasern die keinen runden Faserquerschnitt aufzeigen, z.B. trilobal, pentalobal o.ä.

Stützfasern im Sinne der vorliegenden Erfindung sind Fasern, die in einem erfindungsgemäß aufgebauten Produkt den Faserverbund stabilisieren, sodass ein weitestgehend dimensionsstabiles Vlies entsteht. Stützfasern sind homo- oder bikomponente, thermoplastische Polymerfaser, die schmelzbare Anteile aufweisen. Bei Beaufschlagung mit Temperatur schmelzen diese Anteile und stabilisieren nach dem Erkalten den zunächst nur lose verfestigten Faserverbund. Erfindungsgemäß geeignete Fasern sind homopolymere Stapelfasern aus Co-Polyester, Co-Polyamid oder Polypropylen, erfindungsgemäß bevorzugt werden bikomponente Schmelzfasern in Kern-Mantel oder Side-by-Side Anordnung aus Kombinationen von niedrigschmelzendem Co-Polyester mit Polyester, Polyethylen mit Polypropylen oder Polyethylen mit Polyester. Die Faserfeinheiten liegen im Bereich von 2,2 bis 6,7 dtex, bevorzugt 3,3 bis 4,8 dtex; die eingesetzten Faserlängen sind im Bereich von 10 - 80mm, bevorzugt 35-60mm

Die Anforderungen an Aufnahme- und Verteilvliesstoffe in persönlichen Hygieneartikeln sind neben einer schnellen Flüssigkeitsaufnahme und -verteilung auch eine gute Dimensionsstabilität bei der Verarbeitung und eine geringe Zusammendrückbarkeit im Gebrauch aufweisen.

Gerade bei Druckbelastung darf die in der Aufnahme- und Verteillage zwischengespeicherte Flüssigkeit nicht unkontrolliert abgegeben werden. Es könnte ansonsten zu einem ungewollten Rücknässen kommen.

Dem Stand der Technik entsprechende Aufnahme- und Verteilvliese sind beispielsweise thermisch mittels eines Kalanders gebunden - nachteilig ist dabei dass derartige Vliese aufgrund der Verfestigungsart stark komprimiert sind, somit ein Eindringen von Flüssigkeit erschwert ist. Zudem können nach Quellung der Saugfasern die Bindepunkte aufbrechen und die Stabilität des Vlieses einschränken. Wird als Verfestigungsart die Wasserstrahlverfestigung gewählt, ist zwar eine offene Vliesstruktur die Folge, allerdings sind derartige Vliese leicht zusammendrückbar, sodass unter Belastung darin gespeicherte Flüssigkeit ausgepresst wird. Sind die Vliese chemisch mittels Bindemitteln gebunden, wird ein Großteil der eigentlich zur Flüssigkeitsspeicherung vorgesehenen Fasern durch den Binder verklebt und steht nicht mehr zur Speicherung bereit.

Hier setzt die Erfindung ein. Ein erfindungsgemäß ausgeführtes Vlies weist eine dem Stand der Technik überlegene Resistenz gegenüber Kompressionsbeanspruchung und Flüssigkeitsrückhaltung unter Belastung auf, ohne dass die Flüssigkeitsaufnahme und Verteileigenschaften negativ beeinflusst werden.

Erreicht wird dies durch die Kombination der eingangs erwähnten Faserarten Saugfaser, Stützfaser und Verteilfaser. Die Offenheit des Vliesstoffes und damit die Fähigkeit auftreffende Flüssigkeit schnell aufzunehmen und über die Fläche des erfindungsgemäßen Vliesstoffes zu verteilen wird durch die Verwendung von Verteilfaser gewährleistet.

Die Zwischenspeicherung von aufgetroffener Flüssigkeit wird durch die Saugfaser und die erfindungsgemäße Vliesstruktur gewährleistet.

Die mechanische Stabilität und damit auch die Flüssigkeitsrückhaltung unter Belastung, sowie ein möglichst geringer Unterschied zwischen der Vliesdicke im trockenen und im nassen Zustand, wird über die homogen in der Vliesmatrix verteilten Stützfasern erreicht. Durch das über thermische Aktivierung ausgelöste Schmelzen dieser Fasern bzw. Anteile der Fasern wie dem Schmelzmantel, verbinden sich die Fasern an den Kreuzungspunkten untereinander bzw. mit den Saug- und Verteilfasern.

Nach dem Erkalten bildet sich ein mechanisch stabiles Stützgerüst aus, welches die Parameter "Flüssigkeitsspeicherung unter Belastung" und "Flüssigkeitsrückhaltung unter Belastung" positiv beeinflusst und somit zu einem, dem Stand der Technik überlegenen Material führt.

Hergestellt werden erfindungsgemäße Vliesstoffe mittels Trocken- oder Nassverfahren, die zugrundeliegenden Verfahren können dem im Jahr 2000 bei Wiley VCH-Verlag, Weinheim erschienenen Buch "Vliesstoffe" entnommen werden. Die nachfolgenden Beispiele beziehen sich, ohne darauf beschränkt zu sein, auf nach einem Trockenverfahren unter Verwendung des Kardierverfahrens hergestellte Stapelfaservliesstoffe. Zur Anwendung kamen als Verfestigungsverfahren das erfindungsgemäße Wasserstrahlverfahren in Kombination mit einer erfindungsgemäß nachgeschalteten Heißluftverfestigung mit dem Ziel einer Produktdichte von kleiner 100 kg/m$^3$ und zur Verdeutlichung des Stands der Technik auch die Kalanderverfestigung, sowie zweilagige Materialien.

Die drei erfindungsgemäß zu verwendenden Fasertypen werden dabei zunächst unter Einsatz klassischer Ballenöffnungs- und Mischeinrichtungen miteinander homogen vermischt und der Vliesbildungseinrichtung zugeführt.

Die Mischungsverhältnisse der drei Komponenten zueinander, d.h. der Anteil an Saug-, Verteil- und Stützfaser richtet sich dabei nach dem Anforderungsprofil für den gedachten Endeinsatzzweck.

Die genaue Zusammensetzung des erfindungsgemäßen Aufnahme- und Verteilvliesstoffes ist dabei anhand des jeweiligen Anforderungsprofiles an das Endprodukt zu ermitteln. Erfindungsgemäß sind dabei Anteile von 25-35% an Stützfasern, 35-80% an Verteilfasern und 20-50% Saugfasern vorgesehen, die entsprechend für die jeweilige Anwendung wie nachstehend beschrieben zu einander variiert werden können. Dies bei Flächengewichten des erfindungsgemäßen Produkts von 25-120 g/m$^2$.

Soll beispielsweise der Aufnahme- und Verteilvliesstoff im Bereich von Windeln Anwendung finden, so ist eine höhere Aufnahme- und Verteilwirkung gefordert, da pro Zeiteinheit im Vergleich zu beispielsweise einer Slipeinlage wesentlich mehr Flüssigkeit aufgenommen werden muss, auch ist eine höhere mechanische Stabilität gefordert. Entsprechend höher ist der Anteil an Verteilfasern und Stützfasern zu setzen, sinnvolle Flächengewichte liegen zwischen 40 und 100g/m$^2$.

Werden Windeln für Babys mit einem erfindungsgemäßen Aufnahme- und Verteilvliesstoff ausgestattet, so ist das Hauptaugenmerk mehr auf die Flüssigkeitsaufnahme und Verteilwirkung zu legen. Entsprechend reduziert wird der Anteil an Stützfasern. Erfindungsgemäß haben sich Mischungen mit einem Anteil von 30-35% Stützfasern, 35-40% Verteilfasern mit einem Titer im Bereich von 10-12 dtex und 25-35% trilobalen Saugfasern bewährt.

Wird das erfindungsgemäße Aufnahme- und Verteilvlies bei einem Produkt für die Erwachseneninkontinenz eingesetzt, ist die Flüssigkeitsrückhaltung unter Belastung das Hauptkriterium bei der Bewertung. Hier wird der Anteil an Stützfasern und Saugfasern erhöht, der Anteil an Verteilfasern liegt vergleichsweise gering. Hier kommen erfindungsgemäß Mischungen aus 30- 35% Stützfasern, 25-30% Verteilfasern mit einem Titer im Bereich von 10-12 dtex und 35-40% trilobalen Saugfasern zum Einsatz.

Ist der Anwendungsbereich des erfindungsgemäßen Produkts im Bereich von Slipeinlagen zu sehen, so sind Flächengewichte von 25-70 g/m$^2$ anzustreben. Aufgrund der Tatsache, dass hier die Immobilisierung von Körperexudaten bei gleichzeitig weicherem Griff im Vordergrund steht, setzt man hier Verteilfaser mit einer Faserfeinheit von 10dtex oder weniger ein. Des Weiteren werden ebenfalls geringere Anteile an Stützfasern eingesetzt mit der Absicht, einen weichen Griff im Verteilvlies zu erreichen. Für diesen Einsatzzweck kommen daher erfindungsgemäß Mischungen aus 25-30% Stützfasern, 30-40% Verteilfasern mit einem Titer im Bereich von 4,4-6,7dtex und 30-45% Saugfasern aus normaler Viskosefaser zum Einsatz.

In den folgenden Ausführungen wird näher auf erfindungsgemäße Materialien eingegangen, die als Verteilvliese für die Einsatzbereiche Hygieneeinlagen zur Speicherung von Körperflüssigkeiten wie z.B. Inkontinenzeinlagen, Binden und Pantilinern (erfindungsgemäßes Vlies 1), Babywindeln (erfindungsgemäße Vliese 2 und 3) und Erwachsenenwindeln (erfindungsgemäßes Vlies 4) geeignet sind. Verglichen werden diese mit Vergleichsbeispielen 1, 2 und 3, die den Stand der Technik wiederspiegeln.

Ergänzend dazu sind in Tabelle 1 die einzelnen erzielten Prüfergebnisse aufgeführt. Die dabei ermittelten Parameter wurden gemäß den nachfolgend genannten Prüfmethoden ermittelt:

- Flächengewicht gemäß WSP 130.1
- Maximale Wasseraufnahme gemäß WSP 10.1, wobei die Berechnung anhand der nachstehenden Formel geschieht:

$$\text{Maximale Wasseraufnahme } [\%] = \frac{AW\,[g] - EW\,[g]}{EW\,[g]} * 100\%$$

AW: Gewicht des mit Flüssigkeit getränkten Vlieses
EW: Gewicht des trockenen Vlieses

- Steighöhe gemäß WSP 10.1
- Gewichtsbezogene Flüssigkeitsrückhaltung bei Belastung gemäß WSP 242.2(09), gemäß dieser Norm wird mit einer Mustermasse von 1 g gearbeitet. Die Masse des Prüflings wird zu Beginn des Tests bestimmt und als "Masse vor Belastung 1" M1 verzeichnet.

Dieses Muster wird zum lastfreien Saugen für 5 Minuten in destilliertes Wasser gelegt. Der Prüfling muss dabei vollständig im Wasser eingetaucht sein. Nach Ablauf der 5 Minuten wird das Vliesmuster gefaltet und in einen Zylinder mit perforiertem Boden gelegt und für 2 Minuten mit einem Gewicht beaufschlagt, sodass sich ein Druck von 0,3515 N/m$^2$ ergibt. Abschließend wird das Gewicht des Prüflings erneut ermittelt und als "Masse nach Belastung 2" M2 verzeichnet.

Die gewichtsbezogene Flüssigkeitsrückhaltung bei Belastung, GFRbB errechnet sich dann nach folgender Formel:

$$GFRbB\ [\%] = \frac{M2\ [g] - M1[g]}{M1\ [g]} * 100$$

- Flächenbezogene Flüssigkeitsrückhaltung bei Belastung in Anlehnung an WSP 242.2 (09), abweichend zu dieser Norm wird nicht mit 1g Mustermenge gearbeitet sondern es wird ein Kreismuster mit Durchmesser 51 mm verwendet. Das Gewicht des Prüflings ergibt sich dabei anhand des Flächengewichts des zu untersuchenden Vliesstoffes. Bei einem Flächengewicht von 100g/m$^2$ hat dann ein Prüfling eine Masse von 0,204g. Die Masse des Prüflings wird zu Beginn des Tests bestimmt und als "Masse vor Belastung 3" M3 verzeichnet. Dieses Muster wird zum lastfreien Saugen für 5 Minuten in destilliertes Wasser gelegt. Der Prüfling muss dabei vollständig im Wasser eingetaucht sein. Nach Ablauf der 5 Minuten wird das Vliesmuster in einen Zylinder mit perforiertem Boden gelegt und für 2 Minuten mit einem Gewicht beaufschlagt, sodass sich ein Druck von 0,3515 N/m$^2$ ergibt. Abschließend wird das Gewicht des Prüflings erneut und als "Masse nach Belastung 4" M4 verzeichnet.

Die flächenbezogene Flüssigkeitsrückhaltung bei Belastung, FFRbB errechnet sich dann nach folgender Formel:

$$FFRbB\ [\%] = \frac{M4\ [g] - M3[g]}{M3\ [g]} * 100$$

- Gewichtsbezogene Flüssigkeitsaufnahme unter Belastung in Anlehnung an WSP 242.2 (09), abweichend zu dieser Norm lässt man das Muster nicht lastfrei saugen, sondern unter Belastung von 0,3515 N/m$^2$.
  Gemäß dieser Norm wird mit einer Mustermasse von 1 g gearbeitet.
  Die Masse des Prüflings wird zu Beginn des Tests bestimmt und als "Masse vor Belastung 5" M5 verzeichnet.
  Dieses Muster wird dann gefaltet und auf den perforierten Boden des Test-Zylinders gelegt, anschließend mit einem Gewicht belastet, sodass sich ein Druck von 0,3515 N/m$^2$ ergibt. Der so entstandene Testaufbau wird dann für 5 Minuten in destilliertes Wasser getaucht. Der Prüfling muss dabei vollständig im Wasser eingetaucht sein. Nach Ablauf dieser Zeit wird der Test-Zylinder aus dem Wasserbad entnommen. Danach wird das Vliesmuster aus dem Zylinder entnommen und für 2 Minuten abgetropft. Abschließend wird das Gewicht des Prüflings erneut bestimmt und als "Masse nach Belastung 6" M6 verzeichnet.
  Die gewichtsbezogene Flüssigkeitsaufnahme bei Belastung, GFAbB errechnet sich dann nach folgender Formel:

$$GFAbB\ [\%] = \frac{M6\ [g] - M5[g]}{M5\ [g]} * 100$$

- Flächenbezogene Flüssigkeitsaufnahme unter Belastung in Anlehnung an WSP 242.2 (09), abweichend zu dieser Norm wird nicht mit 1g Mustermenge gearbeitet sondern es wird ein Kreismuster mit Durchmesser 51 mm verwendet. Das Gewicht des Prüflings ergibt sich dabei anhand des Flächengewichts des zu untersuchenden Vliesstoffes. Bei einem Flächengewicht von 100g/m$^2$ hat dann ein Prüfling eine Masse von 0,204g.
  Die Masse des Prüflings wird zu Beginn des Tests bestimmt und als "Masse vor Belastung 7" M7 verzeichnet.
  Dieses Muster wird dann auf den perforierten Boden des Test-Zylinders gelegt und mit einem Gewicht belastet, sodass sich ein Druck von 0,3515 N/m$^2$ ergibt. Der so entstandene Testaufbau wird dann für 5 Minuten in destilliertes Wasser getaucht. Der Prüfling muss dabei vollständig im Wasser eingetaucht sein.
  Nach Ablauf dieser Zeit wird der Test-Zylinder aus dem Wasserbad entnommen. Danach wird das Vliesmuster aus dem Zylinder entnommen und für 2 Minuten abgetropft. Abschließend wird das Gewicht des Prüflings erneut und als "Masse nach Belastung 8" M8 verzeichnet.
  Die flächenbezogene Flüssigkeitsrückhaltung bei Belastung, FFAbB errechnet sich dann nach folgender Formel:

$$FFAbB\ [\%] = \frac{M8\ [g] - M7[g]}{M7\ [g]} * 100$$

- Trockendicke in Anlehnung an WSP 120.6. Abweichend davon wird einem Messdruck von 0,03515 N/m$^2$ gearbeitet
- Nassdicke in Anlehnung an WSP 120.6. Das Prüfmuster wird eine Minute in 0,9%-ige NaCL-Lösung eingelegt, dann wird das Muster eine Minute abgetropft, unmittelbar daran anschließend erfolgt die Messung bei einem Messdruck von 0,03515 N/m$^2$.
- Strike Through gemäß WSP 70.7
- Rewet gemäß WSP 70.8
- Run off in Anlehnung an WSP 80.9, abweichend zu dieser Norm wird mit 75 ml statt 50 ml NaCl-Lösung gearbeitet, die schiefe Ebene hat ein Gefälle von 25° statt 45°. Die Berechnung des RunOff erfolgt dabei nach nachfolgender Formel:

$$\text{Run off } [\%] = \frac{Run\ off\ Menge\ in\ g}{Aufgebrachte\ Flüssigkeitsmenge\ in\ g} * 100$$

Der erfindungsgemäß ausgeführte Vlies 1 stellt ein Verteilvlies dar, welches in einer Hygiene Einlage zur Speicherung von Körperflüssigen (Inkontinenzeinlage, Binde, Pantiliner) zum Einsatz kommen soll. Gemäß den vorstehenden Ausführungen ist eine Immobilisierung der auftreffenden Flüssigkeit bei gleichzeitig weichem Griff die Vorgabe.
Das erfindungsgemäß ausgeführte Vlies 1 besteht aus einer Mischung von

- 40% Verteilfasern, gebildet aus handelsüblichen hydrophilen Polyethylenterephthalatfasern mit einem Titer von 6,7dtex, Stapellänge 60mm
- 25% Stützfasern, gebildet aus einer hydrophilen Polyethylenterephthalat / Co-Polyethylenterephthalat-Faser in einem Titer von 4,4dtex, Stapellänge 51 mm
- 35% Saugfasern, gebildet aus einer handelsüblichen, hydrophilen Viskosefaser mit einem Titer von 1,7dtex und einer Stapellänge von 40mm

Die Fasern werden homogen miteinander vermischt und mittels eines Kardierverfahrens zu einem Faserflor geformt. Dieser Faserflor wird einer Wasserstrahlvernadelungsanlage der Bauart Fleissner mit einer Vorvernadelung und vier Vernadelungsbalken vorgelegt. Auf den jeweiligen Vernadelungstrommeln waren mikroporöse Schalen angeordnet. Die Wasserstrahlen werden mit Düsenstreifen eines Lochdurchmessers von 0,12mm erzeugt.
Das so vorverfestigte Vlies wird einem nachfolgenden Wärmeprozess zur Aktivierung der schmelzbaren Anteile innerhalb der erfindungsgemäß vorhandenen Stützfasern zugeführt. Abhängig vom Typ der eingesetzten Stützfasern wird das Vlies auf Temperaturen oberhalb des Schmelzpunktes der schmelzbaren Anteile der Stützfasern erhitzt. Diese Temperatur müssen für ca. 30 Sekunden im Vlies zur Gewährleistung eines ausreichenden Schmelzflusses der schmelzbaren Anteile erreicht werden. Im Falle des erfindungsgemäßen Vlieses 1 lag die Temperatur bei 105 °C.
Abschließend wird das so erhaltene Produkt abgekühlt und kann entsprechend den Anforderungen an die Weiterverarbeitung konfektioniert werden. Das so erzeugte, erfindungsgemäße Vlies 1 hat ein Flächengewicht von 70 g/qm.
Ein dem Stand der Technik entsprechendes Aufnahme - und Verteilvlies, in der Tabelle 1 als Vergleichsbeispiel 2 aufgeführt, wird durch Mischung aus 80% einer trilobalen Viskosefaser im Titer von 3,3dtex/38mm mit 20% einer bikomponenten Stützfaser aus Polyethylen im Mantel und Polethylenterephthalat im Kern mit einem Titer von 2,2dtex/51mm gebildet. Diese Fasermischung wird kardiert und einem Kalander zur thermischen Verfestigung zugeführt. Das resultierende Vliesgewicht liegt bei 100 g/m$^2$.
Betrachtet man nun die Tabelle 1 so zeigt sich, dass das erfindungsgemäß ausgeführte Vlies 1 trotz einem 30% geringeren Vliesgewicht einen um den Faktor 5 verbesserten Rewet aufweist. Dies wird auch durch die Messwerte für die flächenbezogene Flüssigkeitsrückhaltung unter Belastung bestätigt. Hier zeichnet sich das erfindungsgemäß ausgeführte Produkt mit einem Ergebnis von 1145% gegenüber dem Stand der Technik mit 770% aus.
Dies bedeutet für das erfindungsgemäß ausgeführte Produkt eine um 48% verbesserte Flüssigkeitsrückhaltung bei einer gleichzeitigen Materialeinsparung um 30%, jeweils im Vergleich zu Vergleichsbeispiel 2.
Betrachtet man das erfindungsgemäß ausgeführte Vlies 4 welches in Babywindeln eingesetzt werden soll.
Gemäß den vorstehenden Ausführungen ist ebenfalls eine rasche Flüssigkeitsverteilung der auftreffenden Flüssigkeit gewünscht und Flüssigkeitsspeicherung gewünscht.
Das erfindungsgemäß ausgeführte Vlies 4 besteht aus einer Mischung von

- 35% Verteilfasern, gebildet aus spiralgekräuselten, hydrophilen Polyethylenterephthalatfasern-Hohlfasern mit einem Titer von 10dtex, Stapellänge 38mm
- 25% Stützfasern, gebildet aus einer hydrophilen Polyethylenterephthalat / Co-Polyethylenterephthalat-Faser in einem Titer von 4,4dtex, Stapellänge 51mm

- 40% Saugfasern, gebildet aus einer handelsüblichen, hydrophilen Viskosefaser mit einem Titer von 1,7dtex und einer Stapellänge von 40mm

Die Fasern werden homogen miteinander vermischt und mittels eines Kardierverfahrens zu einem Faserflor geformt. Dieser Faserflor wird einer Wasserstrahlvernadelungsanlage der Bauart Fleissner mit einer Vorvernadelung und vier Vernadelungsbalken vorgelegt. Auf den jeweiligen Vernadelungstrommeln waren mikroporöse Schalen angeordnet. Die Wasserstrahlen werden mit Düsenstreifen eines Lochdurchmessers von 0,12mm erzeugt.

Das so vorverfestigte Vlies wird einem nachfolgenden Wärmeprozess zur Aktivierung der schmelzbaren Anteile innerhalb der erfindungsgemäß vorhandenen Stützfasern zugeführt. Abhängig vom Typ der eingesetzten Stützfasern wird das Vlies auf Temperaturen oberhalb des Schmelzpunktes der schmelzbaren Anteile der Stützfasern erhitzt. Diese Temperatur müssen für ca. 30 Sekunden im Vlies zur Gewährleistung eines ausreichenden Schmelzflusses der schmelzbaren Anteile erreicht werden. Im Falle des erfindungsgemäßen Vlieses 4 lag die Temperatur bei 120 °C.

Abschließend wird das so erhaltene Produkt abgekühlt und kann entsprechend den Anforderungen an die Weiterverarbeitung konfektioniert werden. Das so erzeugte, erfindungsgemäße Vlies 4 hat ein Flächengewicht von 100g/m$^2$.

Ein dem Stand der Technik entsprechendes Aufnahme - und Verteilvlies, in der Tabelle 1 als Vergleichsbeispiel 1 aufgeführt, wird durch Mischung aus 75% einer hydrophilen Polyethylenterephthalat-Faser mit einem Titer von 6,7dtex/60mm mit 25% einer bikomponenten Stützfaser aus Co-Polyethylenterephthalat im Mantel und Polyethylenterephthalat im Kern mit einem Titer von 2,2dtex/51mm gebildet. Diese Fasermischung wird kardiert und einer Wasserstrahlvernadelungsbehandlung wie vorstehend zugeführt. Das resultierende Vliesgewicht liegt bei 60 g/m$^2$.

Betrachtet man nun die Tabelle 1 so zeigt sich, dass das erfindungsgemäß ausgeführte Vlies 4 gegenüber dem Vergleichsbeispiel 1 eine um den Faktor 5 bis 10 verbesserte Flüssigkeitsverteilung, ausgedrückt über die Steighöhe und Steiggeschwindigkeit aufweist. Gleichzeitig ist auch der Runoff entsprechend verringert worden.

Will man nun noch eine verbesserte Flüssigkeitsrückhaltung und damit Zwischenspeicherung im erfindungsgemäßen Produkt erreichen, so kann man bei der Saugfaser weg von normalen Viskosefasern hin zu langsam flüssigkeitsaufnehmenden Typen wie beispielsweise trilobalen Fasern oder auch Tencel / Lyocell-basierenden Typen gehen.

Die erfindungsgemäß ausgeführten Vliese 2 und 3 sind dafür Vertreter.

Das erfindungsgemäß ausgeführte Vlies 2 besteht aus einer Mischung von

- 40% Verteilfasern, gebildet aus spiralgekräuselten, hydrophilen Polyethylenterephthalatfasern-Hohlfasern mit einem Titer von 10dtex, Stapellänge 38mm
- 25% Stützfasern, gebildet aus einer hydrophilen Polyethylenterephthalat / Co-Polyethylenterephthalat-Faser in einem Titer von 4,8dtex, Stapellänge 51 mm
- 35% Saugfasern, gebildet aus einer trilobalen, hydrophilen Viskosefaser mit einem Titer von 3,3dtex und einer Stapellänge von 40mm

Die Fasern werden homogen miteinander vermischt und mittels eines Kardierverfahrens zu einem Faserflor geformt. Dieser Faserflor wird einer Wasserstrahlvernadelungsanlage der Bauart Fleissner mit einer Vorvernadelung und vier Vernadelungsbalken vorgelegt. Auf den jeweiligen Vernadelungstrommeln waren mikroporöse Schalen angeordnet. Die Wasserstrahlen werden mit Düsenstreifen eines Lochdurchmessers von 0,12mm erzeugt.

Das so vorverfestigte Vlies wird einem nachfolgenden Wärmeprozess zur Aktivierung der schmelzbaren Anteile innerhalb der erfindungsgemäß vorhandenen Stützfasern zugeführt. Abhängig vom Typ der eingesetzten Stützfasern wird das Vlies auf Temperaturen oberhalb des Schmelzpunktes der schmelzbaren Anteile der Stützfasern erhitzt. Diese Temperatur müssen für ca. 30 Sekunden im Vlies zur Gewährleistung eines ausreichenden Schmelzflusses der schmelzbaren Anteile erreicht werden. Im Falle des erfindungsgemäßen Vlieses 2 lag die Temperatur bei 120°C.

Abschließend wird das so erhaltene Produkt abgekühlt und kann entsprechend den Anforderungen an die Weiterverarbeitung konfektioniert werden. Das so erzeugte, erfindungsgemäße Vlies 2 hat ein Flächengewicht von 70g/m$^2$.

Ein dem Stand der Technik entsprechendes Aufnahme - und Verteilvlies, in der Tabelle 1 als Vergleichsbeispiel 1 aufgeführt, wird durch Mischung aus 75% einer hydrophilen Polyethylenterephthalat-Faser mit einem Titer von 6,7dtex/60mm mit 25% einer bikomponenten Stützfaser aus Co-Polyethylenterephthalat im Mantel und Polyethylenterephthalat im Kern mit einem Titer von 2,2dtex/51mm gebildet. Diese Fasermischung wird kardiert und einer Wasserstrahlvernadelungsbehandlung wie vorstehend zugeführt. Das resultierende Vliesgewicht liegt bei 60 g/m$^2$.

Durch den Einsatz der trilobalen Viskosefaser verringert sich im Vergleich zum erfindungsgemäßen Vlies 4 zwar die Sauggeschwindigkeit auf die Hälfte, jedoch erhöht sich die flächenbezogene Flüssigkeitsspeicherung unter Belastung deutlich. Durch den Austausch der trilobalen Viskosefasertype gegen eine nach dem Lyocell-Verfahren hergestellte Viskose reduziert sich die Geschwindigkeit, dafür bleibt die Dicke im nassen Zustand stabiler was sich positiv auf den Rewet auswirkt. Das erfindungsgemäße Vlies 3 ist hierzu ein Beispiel. Es wurde unter Anwendung der gleichen Verfahrensparameter wie das Vlies 2 hergestellt.

Durch die Mischung der einzelnen Viskosetypen untereinander kann hier eine genaue Steuerung und Abstimmung der Aufnahmezeiten, sowie der Flüssigkeitsaufnahme und- rückhaltung unter Belastung auf den jeweiligen Anwendungszweck geschehen.

Tabelle 1: Testergebnisse Fluidhandling verschiedene Materialien

| Material | | Vergleichs-beispiel 1 | Erfindungsgemäßes Vlies 1 | Erfindungsgemäßes Vlies 2 | Erfindungsgesäßes Vlies 3 | Vergleichsbeispiel 2 | Erfindungsgemäßes Vlies 4 |
|---|---|---|---|---|---|---|---|
| Einsatzbereich | | Babywindel | Pantiliner | Babywindel | Babywindel | Pantiliner | ErwachsenenInkontinenz |
| Mischung: | | 75% PET 6,7 dtex 60 mm, 25% PET/CoPET 4,8 dtex/51mm | 40% PET 6,7 dtex/ 60mm 25% PET/CoPET 4,4 dtex / 51 mm, 35% normale CV 1,7 dtex/ 40mm | 40% PET 10 dtex / 38mm spiralgekräuselt hohl 25%PET/CoPET 4.8 dtex/ 55mm 35% trilobale CV 3,3 dtex/38mm | 40% PET 10 dtex/ 38mm spiralgekräuselt hohl 25% PET/CoPET 4,8 dtex/ 55mm 35% CV Tencel 3,3 dtex /60 mm | 80 % trilobale CV 3,3 dtex/38 mm 20% PET/PE 2,2 dtex/51 mm | 40% normale CV 1,7 dtex/40mm 25%PET/CoPET 4,4 dtex/51mm 35%PET 10 dtex/ 38mm spiral hohl |
| FG | [g/m$^2$] | 60 | 70 | 70 | 70 | 100 | 100 |
| Max. WA | [%] | 1278 | 1088 | 1323 | 1228 | 1051 | 1118 |
| Steighöhe MD n. 120 sec | [mm] | 8,3 | 23 | 21 | 11,7 | 67,2 | 38,3 |
| Steighöhe CD n. 120 sec | [mm] | 3 | 20 | 17 | 7 | 65,2 | 29,5 |
| Steiggeschwindigkeil MD | [mm/min] | 4,15 | 11,5 | 10,5 | 5,85 | 33,6 | 19,15 |
| Steiggeschwindigkeil CD | [mm/min] | 1,5 | 10 | 8,5 | 3,5 | 32,6 | 14,75 |
| Gewichtsbezogene Flüssigkeitsaufnahme unter Belastung | [%] | 1212 | 1049 | 1245 | 1143 | 824 | 1031 |
| Flächenbezogene Flüssigkeitsaufnahme unter Belastung | [%] | 1499 | 1145 | 1521 | 1382 | 770 | 991 |
| Gewichtsbezogene Flüssigkeitsrückhaltung bei Belastung | [%] | 1048 | 1102 | 1179 | 1157 | 910 | 1239 |
| Flächenbezogene Flüssigkeitstückhaltung bei Belastung | [%] | 1209 | 1020 | 1248 | 1170 | 1019 | 871 |
| Trockendicke | [mm] | 0,91 | 1,15 | 1,22 | 1,28 | 1,39 | 1,76 |

EP 2 692 321 B1

(fortgesetzt)

| Material | | Vergleichs-beispiel 1 | Erfindungsgemäßes Vlies 1 | Erfindungsgemäßes Vlies 2 | Erfindungsgesäßes Vlies 3 | Vergleichsbeispiel 2 | Erfindungsgemäßes Vlies 4 |
|---|---|---|---|---|---|---|---|
| Nassdicke | [mm] | 0,92 | 0,96 | 1,09 | 1,32 | 1,34 | 1,53 |
| Dichte | [g/cm³] | 0,07 | 0,06 | 0,06 | 0,05 | 0,07 | 0,06 |
| 1. Strike Through | [sec] | 0,41 | 0,8 | 0,3 | 0,88 | 1,17 | 0,8 |
| 2. Strike Through | [sec] | 0,83 | 1,5 | 1,3 | 1,16 | 2,07 | 1,2 |
| 3. Strike Through | [sec] | 1,04 | 1,7 | 1,2 | 1,2 | 2,07 | 0,9 |
| Rewet | [g] | 0,14 | 1,5 | 0,43 | 0,1 | 7,554 | 0,81 |
| Run off 75 ml bel 25° | [%] | 6,53 | 0,87 | 2,98 | 4,98 | 0,99 | 0,21 |

**Patentansprüche**

1. Aufnahme- und Verteilvliesstoff aus Stapelfasern für persönliche Hygieneprodukte, **dadurch gekennzeichnet, dass**

   - der Vliesstoff einen Gehalt von

      - 10 bis 35 Massenprozent einer thermoplastischen, synthetischen Stapelfaser als Stützfaser, <u>wobei die Stützfasern homo- oder bikomponente, thermoplastische Polymerfasern sind, die schmelzbare Anteile aufweisen,</u>
      - 35 bis 80 Massenprozent einer Stapelfaser aus thermoplastischen und/oder duroplastischen Polymeren als Verteilfaser und
      - 10 bis 50 Massenprozent einer Stapelfaser aus regenerierter Zellulose als Saugfaser aufweist und

   - der Vliesstoff mechanisch mittels Wasserstrahlen und thermisch mittels nachgeschalteter Heißluftverfestigung gebunden ist und
   - <u>der Vliesstoff eine Dichte von kleiner 100 kg/m3 aufweist.</u>

2. Aufnahme- und Verteilvliesstoff gemäß Anspruch 1, **dadurch gekennzeichnet, dass** die Stützfaser eine homopolymere Synthesefaser ist.

3. Aufnahme- und Verteilvliesstoff gemäß Anspruch 1, **dadurch gekennzeichnet, dass** die Stützfaser eine bikomponente Synthesefaser ist.

4. Aufnahme- und Verteilvliesstoff gemäß Anspruch 1, **dadurch gekennzeichnet, dass** der Vliesstoff ein Flächengewicht von 25-200g/m$^2$ aufweist.


**Claims**

1. Nonwoven fabric for absorption and distribution, made of staple fibres for personal hygiene products, **characterised in that**

   - the nonwoven fabric has a content of

      - 10 to 35 wt.% of a thermoplastic, synthetic staple fibre as a support fibre, the support fibres being homocomponent or bicomponent thermoplastic polymer fibres that comprise fusible constituents,
      - 35 to 80 wt.% of a staple fibre made of thermoplastic and/or thermosetting polymers as a distribution fibre, and
      - 10 to 50 wt.% of a staple fibre made of regenerated cellulose as an absorbent fibre, and

   - the nonwoven fabric is mechanically bonded by means of water jets and thermally bonded by means of subsequent hot-air bonding, and
   - the nonwoven fabric has a thickness of less than 100 kg/m$^3$.

2. Nonwoven fabric for absorption and distribution according to claim 1, **characterised in that** the support fibre is a homopolymer synthetic fibre.

3. Nonwoven fabric for absorption and distribution according to claim 1, **characterised in that** the support fibre is a bicomponent synthetic fibre.

4. Nonwoven fabric for absorption and distribution according to claim 1, **characterised in that** the nonwoven fabric has a weight per unit area of 25-200 g/m$^2$.

**Revendications**

1.  Non-tissé d'absorption et de répartition en fibres courtes pour des produits d'hygiène personnels, **caractérisé en ce que**

    - le non-tissé présente une teneur

        - de 10 à 35 pourcent en masse d'une fibre courte synthétique, thermoplastique, en tant que fibre liante de support, les fibres liantes de support étant des fibres polymères thermoplastiques, homo- ou bi-composant, qui comportent des fractions fusibles,
        - de 35 à 80 pourcent en masse d'une fibre courte en polymères thermoplastiques et/ou thermodurcissables, en tant que fibre de répartition,
        - de 10 à 50 pourcent en masse d'une fibre courte en cellulose régénérée, en tant que fibres d'aspiration ou absorbante,

    - le non-tissé étant lié mécaniquement au moyen d'un traitement au jet d'eau, et, en aval, thermiquement par solidification à l'air chaud, et
    - le non-tissé présente une densité de moins de 100 kg/m$^3$.

2.  Non-tissé d'absorption et de répartition selon la revendication 1, **caractérisé en ce que** la fibre liante de support est une fibre de synthèse homopolymère.

3.  Non-tissé d'absorption et de répartition selon la revendication 1, **caractérisé en ce que** la fibre liante de support est une fibre de synthèse bi-composant.

4.  Non-tissé d'absorption et de répartition selon la revendication 1, **caractérisé en ce que** le non-tissé présente un poids par unité de surface ou grammage de 25 à 200 g/m$^2$.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69222914
- DE 102006000780
- US 4935022 A
- EP 0397110 A2
- WO 9822066 A1
- WO 9802608 A1
- WO 0148291 A1

- EP 0672774 A2
- WO 9822065 A1
- DE 102007028039 A1
- DE 102008007804 A1
- DE 2226330 A1
- US 20060258250 A1
- US 2003087448 A1